(19) Europäisches Patentamt
European Patent Office
Office européen des brevets

(11) Numéro de publication: **0 019 524**
**A1**

(12) # DEMANDE DE BREVET EUROPEEN

(21) Numéro de dépôt: 80400628.6

(22) Date de dépôt: 09.05.80

(51) Int. Cl.[3]: C 07 H 17/06
A 61 K 31/70

(30) Priorité: 09.05.79 GB 7915955

(43) Date de publication de la demande: 26.11.80 Bulletin 80/24

(84) Etats Contractants Désignés: BE DE FR GB IT

(71) Demandeur: SOCIETE DE RECHERCHES INDUSTRIELLES SORI
50, Avenue des Champs Elysées
F-75008 Paris(FR)

(72) Inventeur: Majoie, Bernard
71 rue Montchapet
F-21000 Dijon(FR)

(74) Mandataire: Clisci, Serge et al,
CABINET BEAU DE LOMENIE 55 rue d'Amsterdam
F-75008 Paris(FR)

(54) **Procédé de préparation de dérivés de flavylium, produits nouveaux obtenus selon ce procédé et leur application en thérapeutique.**

(57) La présente demande a trait à un nouveau procédé de préparation de dérivé de flavylium de formule :

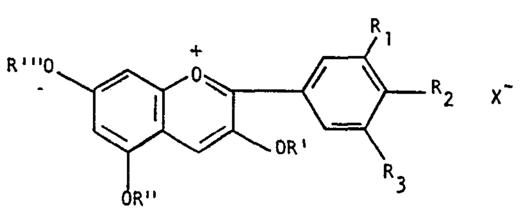
(1)

dans laquelle:

$R_1$, $R_2$ et $R_3$, qui peuvent être identiques ou différents, représentent chacun H, OH, un groupe alkoxy en $C_1$-$C_3$ ou $OCH_2CH_2OH$,

R' représente un radical ose,

R" et R"', qui peuvent être identiques ou différents, représentent chacun H ou un groupe alkyle en $C_1$-$C_3$, R" pouvant en outre représenter un radical ose, et

$X^-$ représente un anion non toxique,

ce procédé comprenant la protection des groupes OH de la flavone correspondante sous forme d'éther de silyle, la réduction du dérivé silyle ainsi obtenu en dérivé silyl-flavylium, puis l'élimination de la protection silyle.

L'invention concerne également les nouveaux produits obtenus selon ce procédé, et leur application en thérapeutique.

EP 0 019 524 A1

Procédé de préparation de dérivés de flavylium, produits nouveaux obtenus selon ce procédé et leur application en thérapeutique.

La présente invention a trait à un nouveau procédé de préparation de dérivés de flavylium. Elle concerne, en tant que produits industriels, les nouveaux dérivés de flavylium obtenus selon ce procédé ainsi que leur application en thérapeutique.

On sait que l'on a déjà préconisé dans le passé des dérivés de flavylium en tant que médicaments soit en raison de leur activité vitaminique P pour améliorer l'acuité visuelle et en particulier la vision nocturne ou pour traiter la fragilité des vaisseaux capillaires (voir à cet effet les brevets britanniques n° 1.007.751, n° 1.068.609 et n° 1.292.794), soit encore en raison de leurs propriétés d'inhiber la rétention du cholestérol dans les artères, d'améliorer la synthèse du collagène et de normaliser la flexibilité et la déformabilité des hématies (voir à cet effet la demande de brevet britannique publiée n° 2.009.159).

Jusqu'à présent, les dérivés de flavylium étaient obtenus soit par extraction de substances naturelles végétales et plus précisément des fruits (cassis, myrtilles, raisins) soit encore par synthèse selon un procédé comprenant la formation d'un dérivé flavénique par réduction catalytique d'une 3-oside-flavone, puis la transformation du dérivé flavénique en sel de flavylium (voir à cet effet le brevet britannique n° 1.292.794 qui décrit à partir de la rutine l'obtention du 3-rhamnoglucosyloxy-3',4',5,7-tétraacétyloxy-3-flavène puis du chlorure de 3-rhamnoglucosyloxy-3',4',5,7-tétrahydroxyflavylium).

Selon l'invention, on préconise un nouveau procédé qui comprend la protection des groupes OH libres au moyen d'un éther de silyle, la réduction du dérivé silyle ainsi obtenu en dérivé silyl-flavylium, puis l'élimination de la protection silyle pour régénérer les groupes OH. Ce nouveau procédé conduit à des rendements plus élevés en sels de flavylium que les procédés antérieurement connus.

Selon l'invention, on propose un nouveau procédé de préparation d'un dérivé de flavylium répondant à la formule générale :

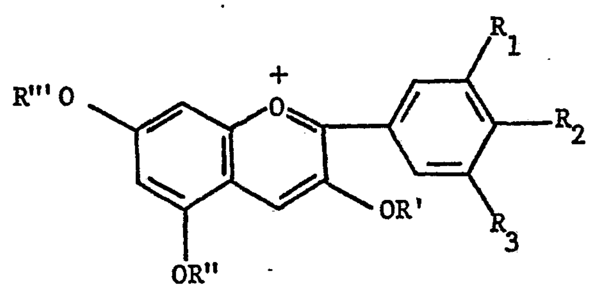

$X^-$ (I)

dans laquelle :

. $R_1$, $R_2$ et $R_3$, qui peuvent être identiques ou différents, représentent chacun H, OH, un groupe alkoxy en $C_1$-$C_3$ ou $OCH_2CH_2OH$,

. R' représente un radical ose,

. R" et R"', qui peuvent être identiques ou différents, représentent chacun H ou un groupe alkyle en $C_1$-$C_3$, R" pouvant en outre représenter un radical ose, et

. $X^-$ représente un anion non toxique minéral ou organique du type $\frac{1}{m} M^{-m}$ [où M est un anion et m sa valence], tel que notamment un anion chlorure, malate, sulfate, tartrate, citrate ou phosphate;

ledit procédé, qui comprend la protection des groupes OH libres et la réduction d'un composé flavonol en flavylium, étant caractérisé en ce que :

a) on fait réagir un agent de silylation avec une flavone de formule :

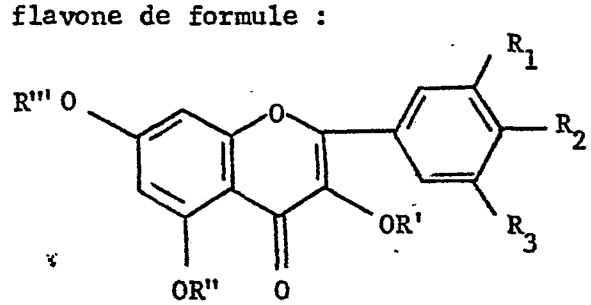

(II)

(où $R_1$, $R_2$, $R_3$, R', R" et R"' sont définis comme ci-dessus),

b) on soumet le dérivé silyle ainsi obtenu à une réaction de réduction pour obtenir un dérivé silylflavylium, et

c) on élimine la protection silyle.

L'agent de silylation du stade a) est notamment choisi parmi les composés donnant des éthers de silyle, tels que le triméthylsilyléther, le diméthyltertiobutylsilyléther, le diméthylisopropylsilyléther, le triisopropylsilyléther, l'éther de silyle préféré étant ici le triméthylsilyléther, ces éthers de silyle sont notamment obtenus à partir des chlorosilanes correspondants. La silylation du stade a) est avantageusement mise en oeuvre dans de la pyridine à 15-50°C pendant 2 à 12 heures, et de préférence pendant 3 heures à 50°C, l'agent de silylation étant en excès par rapport aux conditions stoechiométriques.

La réduction du stade b) est mise en oeuvre dans un solvant inerte tel que le tétrahydrofuranne, le diméthylformamide, le diéthyléther et, d'une manière générale, dans tout solvant aprotique. Le moyen réducteur est notamment choisi parmi l'ensemble constitué par $AlLiH_4$, l'hydrure de diisobutylaluminium, l'hydrure d'aluminium et $(CH_3OCH_2CH_2O)_2AlH_2Na$ - ce dernier produit étant commercialisé sous le nom de marque de "Redal" -. La réduction du stade b) est effectuée en présence d'un excès de moyen réducteur pendant 2 à 4 heures, de préférence pendant 3 heures.

L'élimination de la protection silyle est réalisée au stade c) au moyen d'un acide HX (où X est défini comme ci-dessus).

Après le stade c), le dérivé flavylium de formule (I) peut être avantageusement soumis à une purification comprenant une adsorption sur une résine de polystyrène (pour éliminer un certain nombre d'impuretés) puis, après élution, à une chromatographie sur polyvinylpyrrolidone.

Par l'expression "radical ose", on entend ici un radical dérivant d'un ose tel que notamment le glucose et le rhamnoglucose (ou 6-désoxy-mannoglucose). Parmi les radicaux oses qui conviennent selon l'invention, on peut notamment citer le 6-O-(6-désoxy-α-L-mannopyranosyl)-β-D-glucopyranosyle.

L'anion $X^-$ peut représenter $Cl^-$, 1/3 $PO_4^{3-}$, 1/2 $HPO_4^{2-}$, $H_2PO_4^-$, 1/2 $SO_4^{2-}$, $HSO_4^-$ ou un anion malate, tartrate ou citrate. L'anion préféré est $Cl^-$ , mais les autres, notamment les malate, tartrate et citrate, sont également intéressants du point de vue pharmaceutique.

Parmi les composés inclus dans la définition de la formule (I), on peut notamment mentionner :

- le chlorure de 3-[6-O-(6-désoxy-α-L-mannopyranosyl)-β-D-glucopyranosyloxy]-3',4',5,7-tétrahydroxy-flavylium (composé III),
- le chlorure de 3-[6-O-(6-désoxy-α-L-mannopyranosyl)-β-D-glucopyranosyloxy]-3',4',5,7-tétraméthoxy-flavylium (composé IV).

Selon l'invention, on préconise une composition thérapeutique renfermant, en association avec un excipient physiologiquement acceptable, au moins un composé de formule (I).

On a trouvé que les composés de flavylium de formule (I)
- normalisent la flexibilité et la déformabilité des hématies des patients souffrant de maladies artérielles,
- normalisent le métabolisme du collagène, et
- normalisent la rétention du cholestérol dans les artères, et

qu'ils sont utiles dans le traitement (préventif et curatif) des maladies du coeur et des artères, telles que l'athérome et, d'une manière générale, les angiopathies (c'est-à-dire l'altération de la paroi vasculaire notamment liée à l'hypertension, au diabète et aux autres maladies métaboliques et/ou aux ischémies en résultant).

Plus précisément, les essais qui ont été entrepris montrent que les dérivés de flavylium ont une action remarquable dans le traitement de l'athérome et des angiopathies, en particulier :

- en inhibant la rétention du cholestérol circulant dans les artères : le mécanisme étant probablement celui d'une inhibition de la complexation entre lipoprotéines athérogènes et macromolécules polysaccharidiques tissulaires et cellulaires;

- en normalisant, après traitement, la perturbation de la synthèse du collagène, perturbation qui est initiée par l'hypertension, la plaque graisseuse, le diabète; de cette façon, la synthèse du collagène dans l'aorte du rat hypercholestérolémique, hypertendu ou diabétique (par administration de Streptozotocine), est normalisée au moyen des composés de formule (I); la normalisation ayant lieu au niveau de la quantité de collagène et des différentes fractions solubilisées;

- en améliorant considérablement l'oxygénation dans les pathologies telles que l'athérosclérose, en normalisant la flexibilité des hématies et leur déformabilité; il a été en effet démontré (voir ci-après) que ladite déformabilité est réduite dans les cas pathologiques initiateurs d'athérosclérose : hypercholestérolémie, hypertension, diabète.

Les essais pharmacologiques qui ont été entrepris ont été résumés ci-après.

a) Toxicité

La toxicité a été déterminée chez la souris par voie intrapéritonéale selon la méthode de J.T. LITCHFIELD et al., J. Pharmacol. Exptl. Therap. (1949), 96, 99. La DL-50 I.P. souris des produits III et IV est de 750 mg/kg. Les produits de formule (I) ne sont donc pas toxiques.

b) Inhibition de la rétention du cholestérol

Les essais ont été effectués in vivo sur deux modèles pathologiques de perturbation des paramètres lipidiques initiés chez :

- le rat hyperlipidémique par administration d'un régime gras ayant la composition suivante : 20 parties en poids de caséine, 6 parties en poids d'alphacel, 40 parties en poids de lard, 2 parties en poids de cholestérol, 0,25 partie en poids de chlorure de choline, 1 partie en poids d'acide cholique, 12 parties en poids d'amidon, et des vitamines (traces);

- le rat rendu hypertendu selon la méthode de Goldblatt et al., J. Exp. Med. (1934), 59, 347-379.

Dans chaque cas, les tests ont été effectués sur deux lots de 10 rats chacun (un lot d'animaux traités et un lot d'animaux témoins). Les animaux traités ont reçu le produit à étudier à la dose de 50 mg/kg I.P., à raison de trois administrations par semaine pendant trois semaines.

A la fin de l'expérimentation, les animaux sont sacrifiés, on recueille les aortes, on dose le cholestérol total selon la méthode de C.C. HEUCK et al., J. Lip. Res. (1977), 18, 259, après extraction par chromatographie en phase gazeuse.

Les résultats obtenus montrent la diminution du taux de cholestérol total constaté chez les animaux traités par rapport aux animaux témoins recevant le régime hyperlipidique ou rendus hypertendus.

c) Déformabilité des hématies

La déformabilité des hématies a été mesurée selon la technique de SCHMID-SCHÖNBEIN et al., Blut (1973), 26, 369-379, par filtration (diamètre des pores du filtre : 5 µ) sous une pression constante de 50 mm d'eau, d'une suspension d'hématies amenée à un hématocrite de 10% par dilution dans un plasma autologue. La phase de suspension est également filtrée selon la même technique. On peut apprécier ainsi l'intensité de certaines maladies car le taux de filtration de la suspension d'hématies et/ou de la phase de plasma est modifié par les maladies du métabolisme telles que le diabète et l'hyperlipidémie.

Le taux de filtration des hématies a été déterminé in vivo chez le singe rendu hyperlipidémique après absorption d'un régime gras. Les composés à tester ont été administrés per os à la dose quotidienne de 20 mg/kg pendant 8 mois à des lots de singes : un lot recevant un régime normal, un lot recevant un régime gras, les autres lots recevant le régime gras et un des composés à tester. Les résultats donnés dans le tableau ci-après sont exprimés sous forme d'une moyenne et représentent la vitesse de filtration des hématies ou du plasma en µl/s. La signification statistique des résultats est très bonne ($p < 0,001$).

TABLEAU

| Traitement | Vitesse de filtration du plasma (µl/s) | Vitesse de filtration des hématies (µl/s) |
|---|---|---|
| régime normal (lot de 3 animaux) | 80,34 ± 7,18 | 50,57 ± 5,46 |
| régime hyperlipidique pendant 8 mois (lot de 6 animaux) | 53,58 ± 2,74 | 31,19 ± 5,04 |
| régime hyperlipidique + 20 mg/kg per os de composé III par jour pendant 8 mois (lot de 5 animaux) | 55,92 ± 4,78 | 43,97 ± 2,30 |

Il résulte de ce qui précède que les composés selon l'invention protègent favorablement la paroi artérielle vis-à-vis de l'infiltration lipidique.

D'autres avantages et caractéristiques de l'invention seront mieux compris à la lecture qui va suivre d'exemples de mise en oeuvre nullement limitatifs.

EXEMPLE 1

Préparation du chlorure de 3-[6-O-(6-désoxy-α-L-mannopyranosyl)-β-D-glucopyranosyloxy]-3',4',5,7-tétrahydroxy-flavylium (composé III)

Stade a) : Obtention de la 3-[6-O-(2,3,4-tri-triméthylsilyl-6-désoxy-α-L-mannopyranosyl)-2,3,4-tri-triméthylsilyl-β-D-glucopyranosyloxy]-3',4',5,7-tétra-triméthyl-silyloxy-flavone.

A une solution de 20 g de rutine dans 200 $cm^3$ de pyridine anhydre, on ajoute goutte à goutte 84 $cm^3$ de triméthylchlorosilane à la température ambiante (15-25°C). Après la fin de l'addition du dérivé silyle, on porte le milieu réactionnel à 50°C pendant 3 heures (on observe à cet instant par CCM que toute la rutine

de départ a disparu). La solution ainsi obtenue est évaporée sans pression réduite et le résidu d'évaporation est repris dans de l'éther diéthylique anhydre, on filtre et lave le résidu avec de l'éther diéthylique anhydre. On rassemble les filtrats et les évapore sous pression réduite. Le dérivé silyle qui est obtenu (rendement 40 g) est utilisé tel quel pour la réduction.

Stades b) et c)

On ajoute goutte à goutte 40 g du dérivé silyle du stade a) en solution dans 300 $cm^3$ d'éther à une suspension de 2,4 g de $AlLiH_4$ dans 50 $cm^3$ d'éther. La réduction continue pendant 3 heures. L'excès de $AlLiH_4$ est détruit avec de l'éther saturé en eau, on ajoute 50 $cm^3$ d'eau puis 50 $cm^3$ de HCl 10N. La phase hétérogène est agitée à la température ambiante jusqu'à ce que la coloration rouge de la phase organique ait été transférée en totalité dans la phase aqueuse. On extrait la phase organique avec de l'eau, les phases aqueuses sont rassemblées, extraites avec de l'éther et acétate d'éthyle, les phases organiques ainsi obtenues étant écartées et la phase aqueuse résultante (solution rouge du sel de flavylium dans $H_2O$) est adsorbée sur une colonne de polystyrène (hauteur : 45 cm, diamètre : 7 cm), on lave avec de l'eau distillée et élue avec du méthanol acidifié (les impuretés minérales sont ainsi écartées). L'évaporation de l'éluat sous pression réduite donne un solide rouge (72 g, soit un rendement de 70% par rapport à la rutine de départ) que l'on purifie par chromatographie sur polyvinylpyrrolidone. Le produit résultant est homogène en CCM et en chromatographie sous haute pression, il peut être recristallisé en solution méthanolique par addition d'éther. Rendement 25% par rapport à la rutine.

Propriétés physiques du composé III :

- poudre rouge amorphe,
- pics d'absorption UV : $\lambda$ = 532 nm ( $\varepsilon$ = 19700) et $\lambda$ = 282 nm ( $\varepsilon$ = 11000),
- CCM sur plaque préenduite de cellulose [éluant : $H_2O$-$CH_3COOH$-HCl 12N (82:15:3) v/v], Rf = 0,26.

EXEMPLE 2

On procède comme à l'exemple 1 en remplaçant le stade a) comme suit : à 1 g de rutine dans 25 $cm^3$ de pyridine anhydre, on ajoute goutte à goutte 10 $cm^3$ d'hexaméthyldisilazane et 10 $cm^3$ de triméthylchlorosilane. Après agitation pendant une nuit (12 heures) à la température ambiante (15-25°C), le milieu réactionnel est évaporé sous vide et le résidu est repris dans le tétrachlorure de carbone; les stades b), c) et la purification sont ensuite effectués comme indiqué à l'exemple 1.

EXEMPLE 3

En procédant comme indiqué à l'exemple 1 en remplaçant la rutine par la 3',4',5,7-tétraméthoxy-rutine, on obtient le chlorure de 3-[6-O-(6-désoxy-α-L-mannopyranosyl)-β-D-glucopyranosyloxy]-3',4',5,7-tétraméthoxy-flavylium (composé IV).

Les exemples 4-6 qui suivent ont trait à des compositions thérapeutiques avec un excipient solide ou liquide.

EXEMPLE 4

Le composé actif est mélangé avec de la cellulose, de l'amidon et du stéarate de magnésium. Le mélange obtenu est comprimé de façon à obtenir des comprimés sécables renfermant chacun 10 mg d'ingrédient actif.

EXEMPLE 5

Le composé actif est mélangé avec du lactose et du sucre glace. Le mélange obtenu est granulé par lit fluidisé puis mélangé avec du stéarate de magnésium et comprimé de façon à obtenir des comprimés sublinguaux (glossettes), contenant chacun 10 mg d'ingrédient actif.

EXEMPLE 6

On prépare une composition injectable pour emploi extemporané à partir de 10 mg de composé actif et de 3 ml d'eau pour soluté injectable.

On a résumé enfin les résultats des essais cliniques qui ont été entrepris avec le composé III pendant 21 jours sur 65 patients (30 patients étant hyperlipidiques et les 35 autres patients souffrant d'hypertension artérielle), un lot de patients recevant 3 comprimés ou 3 glossettes par jour renfermant chacun 10 mg de composé III, un autre lot recevant une injection quotidienne de 10 mg de composé III. La tolérance de ce produit s'est révélée excellente puisque la condition de 51 patients (c'est-à-dire 78,4%) a été considérablement améliorée.

# R E V E N D I C A T I O N S

1. Procédé de préparation d'un dérivé de flavylium répondant à la formule générale :

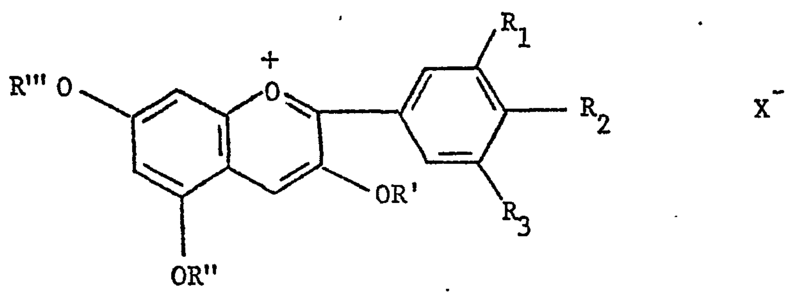

(I)

dans laquelle :

- $R_1$, $R_2$ et $R_3$, qui peuvent être identiques ou différents, représentent chacun H, OH, un groupe alkoxy en $C_1$-$C_3$ ou $OCH_2CH_2OH$,
- R' représente un radical ose,
- R" et R''', qui peuvent être identiques ou différents, représentent chacun H ou un groupe alkyle en $C_1$-$C_3$, R" pouvant en outre représenter un radical ose, et
- $X^-$ représente un anion non toxique,

ledit procédé, qui comprend la protection des groupes OH libres et la réduction d'un composé flavonol en flavylium, étant caractérisé en ce que :

a) on fait réagir un agent de silylation avec une flavone de formule :

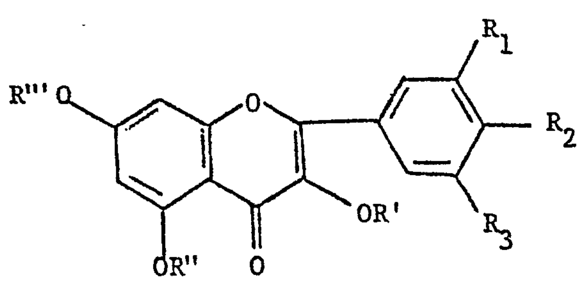

(II)

(où $R_1$, $R_2$, $R_3$, R', R", R''' sont définis comme ci-dessus),

b) on soumet le dérivé silyle ainsi obtenu à une réaction de réduction pour obtenir un dérivé silyl-flavylium, et

c) on élimine la protection silyle.

2. Procédé selon la revendication 1, caractérisé en ce que le stade a) est mis en oeuvre pendant 2 à 12 heures à une température de 15 à 50°C dans de la pyridine anhydre.

3. Procédé selon la revendication 2, caractérisé en ce que le stade a) est mis en oeuvre à une température de 50°C pendant 3 heures.

4. Procédé selon la revendication 1, caractérisé en ce que la réduction du stade b) est mise en oeuvre dans un solvant choisi parmi l'ensemble constitué par le tétrahydrofuranne, le diméthylformamide, le diéthyléther, le moyen réducteur étant choisi parmi l'ensemble constitué par $AlLiH_4$, l'hydrure de diisobutylaluminium, l'hydrure d'aluminium et $(CH_3OCH_2CH_2O)_2AlH_2Na$.

5. Procédé selon la revendication 1, caractérisé en ce que R' est un reste glucosyl ou rhamnoglucosyl, notamment le reste 6-O-(6-désoxy-α-L-mannopyranosyl)-β-D-glucopyranosyle.

6. Procédé selon la revendication 1, caractérisé en ce que $X^-$ est $Cl^-$, 1/3 $PO_4^{3-}$, 1/2 $HPO_4^{2-}$, $H_2PO_4^-$, 1/2 $SO_4^{2-}$ ou $HSO_4^-$.

7. Procédé selon la revendication 1, caractérisé en ce que $X^-$ est un anion malate, tartrate ou citrate.

8. Produit industriel nouveau appartenant à la famille des dérivés de flavylium, caractérisé en ce qu'il est obtenu selon le procédé de l'une quelconque des revendications 1 à 7.

9. Composition thérapeutique, caractérisée en ce qu'elle renferme, en association avec un excipient physiologiquement acceptable, au moins un dérivé de flavylium selon la revendication 8.

Office européen des brevets

**RAPPORT PARTIEL DE RECHERCHE EUROPEENNE**
qui selon la règle 45 de la Convention sur le brevet européen est considéré, aux fins de la procédure ultérieure, comme le rapport de recherche européenne

Numéro de la demande 0019524

EP 80 40 0628

**DOCUMENTS CONSIDERES COMME PERTINENTS**

| Catégorie | Citation du document avec indication, en cas de besoin, des parties pertinentes | Revendication concernée |
|---|---|---|
| | FR - A - 2 029 399 (FERLUX)<br>* Pages 8,9 *<br>-- | 1,9 |
| | FR - A - 2 342 968 (COCA-COLA)<br>* Page 11, lignes 21-33 *<br>-- | 1 |
| | CHEMISCHE BERICHTE, vol. 101, no. 3, 1968, Verlag Chemie GmbH Weinheim, DE<br>E. WITTENBURG "Nucleoside und verwandte Verbindungen,III. Synthese von Thymin-nucleosiden über Silyl-pyrimidin-Verbindungen", pages 1095-1114.<br>* Page 1097 *<br>-- | 1 |
| | CHEMICAL ABSTRACTS, vol. 86, no. 5, 31 janvier 1977, réf. 29577e, page 345.<br>Columbus, Ohio, USA<br>G. FORNI et al. "Use of gas-liquid chromatography for the quantitative analysis of anthocyanidins (example: determination of cyanidol in Sambucus nigra". | ./. |

**CLASSEMENT DE LA DEMANDE (Int Cl.³)**

C 07 H 17/06
A 61 K 31/70

**DOMAINES TECHNIQUES RECHERCHES (Int. Cl.³)**

C 07 H 17/06
A 61 K 31/79
C 07 D 311/62

**RECHERCHE INCOMPLETE**

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes: 1-7
Revendications ayant fait l'objet de recherches incomplètes: 8-9
Revendications n'ayant pas fait l'objet de recherches:
Raison pour la limitation de la recherche: Caracterisation insuffisante des dérivés de flavylium revendiqués comme produits industriels nouveaux ou comme produits actifs dans des compositions pharmaceutiques étant donné ou un produit est nouveau ou ne l'est pas en tant que tel indépendamment de son procédé de préparation.

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

| Lieu de la recherche | Date d'achèvement de la recherche | Examinateur |
|---|---|---|
| La Haye | 16-07-1980 | VERHULST |

OEB Form 1505.1 06.78

Office européen des brevets

**RAPPORT PARTIEL DE RECHERCHE EUROPEENNE**

Numéro de la demande

EP 80 40 0628

-2-

| DOCUMENTS CONSIDERES COMME PERTINENTS | | | CLASSEMENT DE LA DEMANDE (Int. Cl.³) |
|---|---|---|---|
| Catégorie | Citation du document avec indication, en cas de besoin, des parties pertinentes | Revendication concernée | |
| | & Doc. Prep., Journ. Etude Prog. Recents Methodes Anal. Qual., Quant. Struct. Polyphenols Assem. Gen. Groupe - Groupe Polyphenols 1976, 9a, 21 pp.<br>* En entier *<br>-- | | |
| A | CHIMICA THERAPEUTICA, Chimie Thérapeutique, éditions diméo. Arcueil, FR<br>H. POURRAT et al. "Préparation et activité thérapeutique de quelques glycosides d'anthocyanes", pages 33-38.<br>---- | 9 | |
| | | | DOMAINES TECHNIQUES RECHERCHES (Int. Cl.³) |